# EUROPEAN PATENT APPLICATION

(11) **EP 4 768 604 A1**
(43) Date of publication of application: **01.07.2026**
(21) Application number: 24307291.5
(22) Date of filing: 24.12.2024
(51) Int. Cl.: C12Q 1/6886

(54) **METHOD OF DETECTING THE PRESENCE OF EXONS 5 AND 6 OF ANRIL IN A SUBJECT, KIT, SETS OF OLIGONUCLEOTIDES AND THEIR USES**

(71) Applicant: Université de Lorraine, 54000 Nancy (FR); Centre National de la Recherche Scientifique, 75016 Paris (FR)
(72) Inventor: MAENNER, Sylvain, 54000 NANCY (FR); AYADI, Lilia, 54000 NANCY (FR)
(74) Representative: Novagraaf Technologies

(57) **Abstract**

The present invention relates to a method of detecting and/or quantifying the presence of exons 5 and 6 of ANRIL (Antisense Noncoding RNA in the INK4 Locus) in a subject, comprising the following steps of:
1) Preparation of total RNA from a biological sample of the subject,
2) Conversion of RNA molecules into complementary DNA,
3) Loop-mediated isothermal amplification using trans-exonic oligonucleotides that specifically amplify exons 5 and 6,
4) Addition of Cas12a/RNA guides complexes, wherein RNA guide specifically targets exons 5 and 6 by base pairing,
5) Addition of a reporter DNA probe,
6) Detection of cleavage products, wherein detection of cleavage products reveals the presence of exons 5 and 6 of ANRIL, and
7) possibly quantifying expression of exons 5 and 6 of ANRIL.

The present invention also relates to a method for in vitro diagnosis of a cancer in a subject, to a method for in vitro prognosis a cancer in a subject to a kit for implementing the method and to a set of oligonucleotides.

## Description

### Technical field

The present invention refers to a method of detecting the presence of exons 5 and 6 of ANRIL (Antisense Non-coding RNA in the INK4 Locus) in a subject, to a kit and sets of oligonucleotides for implementing the method, and their use.

Therefore, the present invention has utility in medical fields.

In the description below, the references into brackets ([ ]) refer to the listing of references situated at the end of the text.

### Background of the Invention

In humans, multiple pathologies are due to inappropriate regulation of gene expression. The development of strategies aimed at restoring normal gene expression is therefore a major public health issue. These so-called "gene" therapies are complex and require several scientific obstacles to be overcome. One of them lies in the specificity of the therapeutic agent's action. Without specificity, the latter can cause a cascade of undesirable effects.

Non-coding RNAs do not have coding potential, i.e. they are not translated into proteins (e.g. ribosomal or transfer RNAs involved in the translation process of messenger RNAs).

Over the last decade, next generation sequencing approaches highlighted the unexpected diversity of RNAs lacking obvious protein-coding capacity (ncRNAs). The ncRNAs longer than 200-nts are named long non-coding RNAs (IncRNAs). Nowadays, more than 167,000 IncRNAs have been identified in human, many of them being involved in various critical processes including cell proliferation and cell differentiation. The deregulation of the expression of these IncRNAs can therefore affect cell homeostasis and consequently favour the occurrence and/or development of pathologies. They can then be qualified as pathogenic IncRNAs.

According to "the IncRNADisease database", IncRNAs are associated with 529 pathologies divided into several categories, including 3 major ones corresponding to cancers (44.2%), cardiovascular pathologies (11.6%) and neurodegenerative diseases (7.3%). Several IncRNAs are already used as biomarkers as their expression rate correlates with the diagnostic or even prognosis nature of certain pathologies. It is the case of IncRNA PCA3, used as a prognosis biomarker for prostate cancer.

Within the cell, IncRNAs can be located either in the cytoplasm or in the nucleus.

LncRNAs are key regulators of gene expression carrying both cytoplasmic and nuclear functions. Cytoplasmic IncRNAs mainly modulate gene expression by affecting mRNA stability or translation, while nuclear IncRNAs are mostly associated with the genome to regulate gene expression at the chromatin level. The latter implies the activities of epigenetic writers to targeted genomic loci, including for instance the Polycomb group proteins (PcG) composed by the Polycomb repressive complexes 1 and 2 (PRC1 and PRC2). These complexes are responsible for the conversion of euchromatin into heterochromatin by catalyzing the ubiquitylation of histone H2A on lysine 119 (H2AK119Ub) and the trimethylation of histone H3 on lysine 27 (H3K27me3), respectively. Interestingly, multiple IncRNAs have been shown to associate with the PcG and 20% of them are specific PRC2-binders in human cells.

ANRIL (Antisense Noncoding RNA in the INK4 Locus) is one of the IncRNAs associated with PcG activities and several pathologies. It is transcribed from the *9p21* locus in the opposite direction to the *CDKN2A* and *CDKN2B* (cyclin dependent kinase inhibitors 2A and 2B) genes. ANRIL promotes *in cis* the transcriptional silencing of the *CDKN2A* and *B* genes by recruiting the PcG to the *9p21* locus resulting in an increased cell proliferation rate. In addition, ANRIL is expected to modulate *in trans* the expression of genes distant from the *9p21* locus. This is evidenced by the differential expression of more than 200 genes involved in the maintenance of chromatin architecture, cellular proliferation, growth and apoptosis upon the overexpression of ANRIL sub-fragments in HeLa or in HEK293 cells. Expression changes were also observed for 20 genes, implicated in proliferation and apoptosis, upon ANRIL siRNA knockdown in vascular smooth muscle cells (VSMCs). Even though previous studies demonstrated the trans-regulatory activity of ANRIL, the molecular mechanisms involved need to be refined. Sor far, the coding and non-coding genes, which are directly contacted and regulated by ANRIL remain to be investigated in depth.

The *ANRIL* gene consists of 21 exons, with lengths ranging from 74 to 696 nucleotides (mean length of 202 nucleotides). These exons undergo alternative splicing (AS), resulting in the generation of at least 28 different linear isoforms with varying lengths (Cunningham et al., 2022 ([1])) (from 602 to 7713 nts).

ANRIL has undergone extensive analysis employing various methodologies such as RT-qPCR (reverse transcription quantitative polymerase chain reaction), RNA sequencing, or microarrays across numerous cancer cell lines and tissues, and the ANRIL isoforms have been identified in these investigations. The results demonstrated that the vast majority of the linear ANRIL isoforms meaning containing exons 5-6 are upregulated in a wide range of cancer types, including lung (LC), gastric (GC), breast (BC), ovarian (OC), cervical (CC), colorectal (CRC), bladder (BladC), thyroid (TC), brain (BrC), osteosarcoma (OS), myeloma (MM), prostate (PC), leukemia (ATL/AML), melanoma, endometrial (EC), renal (RC), retinoblastoma (RB), head/neck (HNSCC/LSCC), intrahepatic cholangiocarcinoma (iCCA) and hepatocellular (HCC) cancers. Importantly, the overexpression of ANRIL is associated with poor prognosis and a lower overall survival in GC, LC, HCC, HNSCC/LSCC, RC, iCCA, EC, AML, OS, CC and OC (Sanchez et al. : « The Long Non-Coding RNA ANRIL in Cancers", Cancers 2023, 15, 4160. https://doi.org/10.3390/cancers15164160 ([10])). One meta-analysis published in 2022, based on a sample of 1708 cancer patients extracted from 23 studies across 3 databases, has also established a clear correlation between high ANRIL expression, adverse overall survival rates, larger tumor size, advanced TNM stage, and lymph node metastasis (Liu et al., 2022 ([2]). Also, additional studies demonstrated that ANRIL expression is significantly correlated with a higher TNM stage of cancers including LC, GC, HCC, LSCC, OS, CRC, CC, OC, HCC, BladC and OS.

Thus, the ability to detect isoforms of ANRIL from cancer cell lines and tumor samples holds the promise of classifying cancer subtypes based on their aggressiveness.

So far, the standard method widely used for detecting RNA is RT-qPCR (Vijay J. Gadkar and Martin Filion, 2014 ([3])). However, this approach is not ideal for detecting isoforms of ANRIL, for the following main reasons:
1. Weak abundance of ANRIL: Long non-coding RNAs (LncRNAs), including ANRIL, are typically weakly expressed, with estimation at approximately 440 and 2200 copies of ANRIL per HEK293 and NCI-H146 cells, respectively (Alfeghaly et al. « Implication of repeat insertion domains in the trans-activity of the long non-coding RNA ANRIL ». Nucleic Acids Research 49, n° 9 (21 mai 2021): 4954-70 ([4]) and unpublished data). This low abundance necessitates at least 1 µg of total RNA as the starting material for RT-qPCR experiments. Achieving this quantity is challenging, especially when working with samples of tumor origin obtained, for example, through biopsy.
2. Cost: RT-qPCR can be relatively expensive, requiring specialized equipment, thereby contributing to the overall cost.

Together, these points demonstrate that RT-qPCR is not suitable for detecting isoforms of ANRIL.

Thus, a need exists of alternative methods suitable for detecting isoforms of ANRIL, especially from cancer cell lines and tumor samples, so that it can help to classify cancer subtypes based on their aggressiveness.

### Description of the invention

After extensive research, the Applicants have developed an original method, which allows a rapid and reliable method for detecting, at a lower cost, the presence of the vast majority of ANRIL isoforms in a sample. The Applicants named this method TITAN.

These objectives have been achieved due to the selection, by the Applicants, of exons 5 and 6 to be detected, which allows detecting the vast majority of ANRIL isoforms.

Advantageously, the method allows classifying cancers according to how aggressive they are, as the expression of ANRIL, regardless of isoforms, is positively associated with the tumoral aggressiveness of more than 15 cancers.

The Applicants have also succeeded in developing a kit, based on the detection of exons 5 and 6, which represents an additional tool in terms of diagnosis and prognosis of several cancers.

The method of the invention has many advantages:
- it has a very low detection threshold, using as little RNA as possible,
- it is faster than RT-qPCR: around 2h for the method of the invention compared with around 5h for RT-qPCR,
- it has a low cost as it does not necessitate a thermal cycler.

Accordingly, in a first aspect, the present invention provides a method of detecting and/or quantifying the presence of exons 5 and 6 of ANRIL in a subject, comprising the following steps of:
1) Preparation of total RNA from a biological sample of the subject,
2) Conversion of RNA molecules into complementary DNA,
3) Loop-mediated isothermal amplification using trans-exonic oligonucleotides that specifically amplify exons 5 and 6,
4) Addition of Cas12a/RNA guide complexes, wherein RNA guide specifically targets exons 5 and 6 by base pairing,
5) Addition of a reporter DNA probe,
6) Detection of cleavage products, wherein detection of cleavage products reveals the presence of exons 5 and 6 of ANRIL, and
7) Possibly quantifying expression of exons 5 and 6 of ANRIL.

According to the invention, "subject" refers to any mammal, e.g. humans and other primates. The subject may be a healthy subject, or a subject suffering from a pathology, especially a cancer. For example, the cancer may be at least one among lung, gastric, breast, ovarian, cervical, colorectal, bladder, thyroid, brain, osteosarcoma, myeloma, prostate, leukemia, melanoma, endometrial, renal, retinoblastoma, head/neck, intrahepatic cholangiocarcinoma and hepatocellular cancer. More particularly, the cancer is lung cancer.

"Biological sample" refers herein to any material that originates from the subject and that can be studied and analyzed in a laboratory. Taking the sample is not part of step 1). The sample may be for example selected among blood, plasma, serum, cells, tissue, urine, saliva, semen, feces/stool, bone marrow and sputum.

In step 1), the preparation of total RNA may be performed by any method known in the state of the art. It may be, for example, a method selected among Trizol^{™} method, Silica column method, for example using RNeasy mini kit (QIAGEN), alcohol precipitation method, affinity chromatography method, microfluidic method, agarose gel electrophoresis method, and density gradient ultracentrifugation method, this list not being exhaustive.

Step 2) of conversion of RNA, i.e. total RNA, molecules into complementary DNA (cDNA) may be carried out by any method known in the state of the art. It may be, for example, a method selected among Reverse Transcription (RT), Reverse Transcription Polymerase Chain Reaction (RT-PCR), Random Priming, Oligo(dT) Priming, and Target-Specific Priming, this list not being exhaustive. Preferably, the conversion is carried out by Reverse Transcription (RT).

Once complementary DNA is obtained, step 3) of Loop-mediated isothermal amplification (also called "LAMP") is performed, so as to obtain multicopy DNA. This step may be performed according to the method described by Soroka et al., 2021 (« Loop-Mediated Isothermal Amplification (LAMP): The Better Sibling of PCR? » Cells 10, no 8 (29 juillet 2021): 1931 ([5]). If necessary, routine adjustments can be made to this method, according to the general knowledge of those skilled in the art. This technique enables the multiplication of target copies for detection, i.e. exons 5 and 6, to reach a critical detection threshold. Advantageously, it is highly specific, requiring a combination of an optimum of 4 to 5 oligonucleotides designed to hybridize several distinct locations within the target cDNA produced in step 2) (as opposed to only 2 for standard PCR). Furthermore, this technique drastically increases the quantities of amplified DNA of interest (up to 1 billion copies in 1 hour, compared to 1 million for standard PCR, due to the generation of multiple initiation sites during the reaction). Also, this technique does not require sophisticated equipment like a thermocycler. The reaction is carried out at a constant temperature of 65°C due to the intrinsic properties of the Bst DNA polymerase used in the approach described by Soroka et al. ([5]).

According to the invention, LAMP is carried out with trans-exonic oligonucleotides. "Trans-exonic oligonucleotides" refers herein to oligonucleotides, also called "primers", designed to amplify regions of a gene, that span multiple exons. In the present case, these multiple exons mean exon 5 and exon 6. Surprisingly, based on extensive research, the Applicants have understood that using trans-exonic primers is mandatory for producing amplicons that specifically contain exons 5 and 6, significantly reducing the collateral effects of genomic DNA contamination. Indeed, these primers do not bind to intron regions present in genomic DNA, ensuring that any amplification is specific to cDNA. This specificity reduces false positives from genomic DNA contamination in RNA samples, eliminating in addition the need for DNase treatment. It is important to note that this is the first time that trans-exonic oligonucleotides are used in a LAMP amplification.

Advantageously, the trans-exonic oligonucleotides may be at least one chosen among:
- O7: TGTCCCTTTTGATGAGAAGA (SEQ ID NO: 1)
- O8: CCAAATAGATCTCCCCGG (SEQ ID NO: 2)
- O9: ACATATATCTGGTGGCCAGAAAACACCTCATTCTGATTCAACAGC (SEQ ID NO: 3)
- O10: AGGAGAATTTTCTTGGAAAGAGAGGAAAGCAGTACTGACTCGG (SEQ ID NO: 4)
- O11: CATCACTGTTAGGTGTGCTGGAAT (SEQ ID NO: 5).

Advantageously, a combination of 2 to 5, preferably 4 or 5, of these trans-exonic oligonucleotides may be used. The oligonucleotides may be at least one combination chosen among:
- a mix of O9 and O10, and possibly O11, and
- a mix of O7 and O8.

Advantageously, trans-exonic oligonucleotides can specifically produce an amplicon containing exons 5 and 6. According to the invention, "specifically" means that the method produces an amplicon containing exons 5 and 6 at a level of at least 95%, for example 96%, 97%, 98%, 99% or even about 100% of the total number of amplicons formed.

Regarding step 4) of the method, CRISPR/Cas12a system used may be performed according to the method described by Chen et al. ([6]), Broughton et al. ([7]), Sun et al. ([8]) or Paul and Montoya ([9]), also known as "DETECTR method". If necessary, routine adjustments can be made to this method, according to the general knowledge of those skilled in the art. Cas12a and RNA guide may be added separately, and they can form complexes in the reaction medium. As already described in the state of the art, the CRISPR/Cas12a system requires the involvement of two elements: an endonuclease called Cas12a and a guide RNA, also called herein "Cas12a/RNA guide complexes". The latter results from the fusion of two functional RNAs: the crRNA, which facilitates the recognition of a targeted DNA region, which may be for example about 20 nucleotides complementary to the target, and the tracrRNA (5'-UAAUUUCUACUAAGUGUAGAU-3'), which is responsible for associating with Cas12a. The formed ribonucleoprotein (RNP) is capable of specifically binding to the 20-nucleotides targeted DNA region and catalysing the degradation of the targeted region upstream of a Protospacer Adjacent Motif (PAM) sequence of type 5'-TTTN-3'. In addition to this targeted degradation, the Cas12a has an additional activity termed collateral. Once activated, Cas12a also degrades any nearby DNA fragment, regardless of its sequence. In the method of the invention, this "nonspecific" activity is used to cleave a reporter DNA fragment, allowing to indirectly assess the presence of the RNA of interest, i.e. the exons 5 and 6.

In Cas12a/RNA guide complex, i.e., Cas12a complexed with RNA guide, said RNA guide may have any sequence that specifically targets exon 5 and exon 6-containing fragment by base pair pairing. In other words, RNA guide may recognize exons 5 and 6 and hybridize with them. Advantageously, the RNA guide hybridizes on the Exon 5-Exon 6 junction, which provides specific recognition of the amplicons of interest and reduces false positives attributed to genomic DNA contamination in RNA samples. For example, RNA guide may comprise the sequence, or consist of the sequence: UAAUUUCUACUAAGUGUAGAUACAGUCUCUCCAAUGAACGCC (SEQ ID NO: 6). The RNA guide may comprise, on one side or on both sides, sequences to provide stability to the ends of the guide RNA formats, to protect them against degradation by environmental and intracellular nucleases.

The reporter probe may be added in the reaction medium at the same time as the guide RNA and Cas12a. Alternatively, it may be added after the formation of the Cas12a/RNA guide complexes in the reaction medium. The reporter DNA probe may be any DNA sequence. Advantageously, the probe may have a fluorophore at one side, in order to facilitate its detection, and eventually a quencher at the other side, in order to help detecting the probe or reading the results, as is known in the art. For example, the reporter DNA probe may be coupled 5' to 6-Carboxyfluorescein (F) and 3' to biotin (B). For example, the DNA probe may comprise at least one sequence chosen among the sequence TTATTATT, TTATT and TTATTATTATT (SEQ ID NO: 7).

Once the guide RNA forms a complex with Cas12a and directs it to the target sequence, Cas12a undergoes a conformational change that activates its endonuclease activity. When Cas12a is activated and begins cleaving the target DNA, it may also cleave the nearby fluorescent probe because of the Cas12a collateral activity. Then, cleavage of the probe releases the fluorescent reporter molecule, commonly a fluorescent reporter molecule, resulting in a detectable and possibly measurable change in fluorescence signal. Detection may be carried out by means commonly used in the state of the art, for example with anti-fluorescein antibodies, especially on strip, fluorescence spectroscopy, fluorescence plate readers, flow cytometry, gel electrophoresis, this not being exhaustive.

All definitions and explanations of technical features or terms given in relation with the method of detection of the invention apply to the following other objects of the invention.

Another object of the invention relates to a method for in vitro diagnosis of a cancer in a subject, comprising the step of:
- detecting the presence of exons 5 and 6 of ANRIL in a biological sample of a subject with a method as defined above,
   Wherein:
   - a detection of exons 5 and 6 of ANRIL in the sample indicates a presence of ANRIL-related cancer, and,
   - an absence of detection of exons 5 and 6 of ANRIL in the sample indicates an absence of ANRIL-related cancer.

Another object of the invention relates to a method for in vitro prognosis a cancer in a subject, comprising the step of:
- quantifying expression of exons 5 and 6 of ANRIL in a biological sample of a subject with a method as defined above,
Wherein:
- A higher abundance level of exons 5 and 6 of ANRIL compared to a reference value is predictive to a more aggressive cancer, and
- An equivalent or lower level of exons 5 and 6 of ANRIL compared to a reference value is predictive of a less aggressive cancer.

As mentioned previously, the cancer may be at least one among lung, gastric, breast, ovarian, cervical, colorectal, bladder, thyroid, brain, osteosarcoma, myeloma, prostate, leukemia, melanoma, endometrial, renal, retinoblastoma, head/neck, intrahepatic cholangiocarcinoma and hepatocellular cancer. More particularly, the cancer is lung cancer.

"Aggressive cancer" or "more aggressive cancer" refers herein to a cancer that forms, grows, or spreads quickly, or to a cancer which is associated with a short survival time, or to a cancer which is at an advanced stage, for example with presence of metastasis. Although there is no official, uniform definition of tumor aggressiveness, the skilled person knows how to assess this characteristic depending on the type of cancer and their general knowledge.

"Less aggressive cancer" refers herein to the contrary of the aggressive cancer.

"Reference value" refers herein to a predetermined threshold, calculated by common statistical mathematical algorithms and data obtained from a statistically significant sample of people. For example, the reference value may be calculated using a retrospective measurement of the level of exons 5 and 6 of ANRIL of samples from historically classified subjects, i.e. subjects classified as having aggressive cancer and subjects as having less aggressive cancer. Then, Kaplan Meier curves may be made for each of the two subsets, and the p-value between the two subsets can be calculated. The reference value is then selected in such a way that discrimination on the basis of the minimum p-value criterion is strongest. In other words, the level of exons 5 and 6 of ANRIL corresponding to the boundary between the two subsets for which the p-value is minimum is taken as the predetermined reference value. Advantageously, the reference value allows discrimination between a subject who may be considered likely to have an aggressive cancer, and a patient likely to have a cancer which is not considered as aggressive.

Another object of the invention relates to a kit for implementing the method as defined of detection and/or quantification of the invention, comprising the following elements:
- trans-exonic oligonucleotides for specific exons 5 and 6 amplification,
- Reagents for RT-LAMP amplification and Cas12a digestion reactions
- RNA guide specifically targeting exons 5 and 6 by base pairing,
- a reporter DNA probe, and
- a mean for the detection of cleavage products.

Another object of the invention relates to a set of oligonucleotides having at least one sequence chosen among:
- O7: TGTCCCTTTTGATGAGAAGA (SEQ ID NO: 1)
- O8: CCAAATAGATCTCCCCGG (SEQ ID NO: 2)
- O9: ACATATATCTGGTGGCCAGAAAACACCTCATTCTGATTCAACAGC (SEQ ID NO: 3)
- O10: AGGAGAATTTTCTTGGAAAGAGAGGAAAGCAGTACTGACTCGG (SEQ ID NO: 4)
- O11: CATCACTGTTAGGTGTGCTGGAAT (SEQ ID NO: 5).

Advantageously, the set of oligonucleotides may comprise a mix of oligonucleotides chosen among:
- a mix of O9 and O10, and possibly O11, and
- a mix of O7 and O8.

Another object of the invention relates to a kit as defined above, or of a set of oligonucleotides as defined above, for detecting and/or quantifying exons 5 and 6 of ANRIL in a biological sample of a subject.

This invention is further illustrated by the following examples with regard to the annexed drawings that should not be construed as limiting.

### Brief description of the figures

- Figure 1: represents the position on exons 5 and 6 of the trans-exonic primers used for the detection of ANRIL isoforms containing exons 5 and 6.
- Figure 2: represents the lateral flow strip assay readout, showing the results of the detection method. As observed for H146 cells, a positive result requires detection of the ANRIL Exons 5-6 target gene. Right hand column: detection method performed on H146 cells, showing a band on control and a band at ANRIL exons 5-6. Middle column: detection method performed on A549 cells, showing only the control band. Left hand column: control method performed with no RNA, only showing the control band.

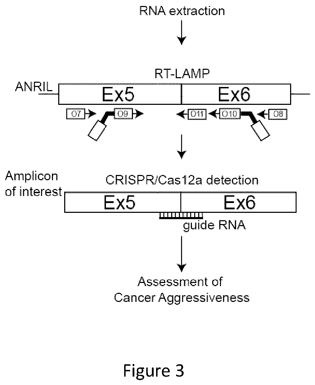

- Figure 3: represents main steps of the method, involving primers O7, O8, O9, O10, O11, and gRNA used for assessment of cancer aggressiveness.

### Examples

### Example 1: Implementation of the method of detecting/quantifying the presence of exons 5 and 6 of ANRIL in a subject

### 1. Primer sequences

### 1.1. Primer sequences used for the detection of ANRIL isoforms containing exons 5-6

Primer sequences (Merck Sigma Aldrich) used are the following:
O7: TGTCCCTTTTGATGAGAAGA (SEQ ID NO: 1), which spans on exon 5
O8: CCAAATAGATCTCCCCGG (SEQ ID NO: 2), which spans on exon 6
O9: ACATATATCTGGTGGCCAGAAAACACCTCATTCTGATTCAACAGC (SEQ ID NO: 3), which spans on exon 5
O10: AGGAGAATTTTCTTGGAAAGAGAGGAAAGCAGTACTGACTCGG (SEQ ID NO: 4), which spans on exons 5 and 6
O11: CATCACTGTTAGGTGTGCTGGAAT (SEQ ID NO: 5), which spans on exon 6. This primer has been specifically designed to hybridize within the amplicon generated by primer O10. Primer O10 is engineered to produce an amplicon that forms a hairpin structure as its 3' extremity, with a single-stranded loop. This loop can serve as a hybridization site for another primer, such as O11. By base-pairing with the loop, O11 creates an additional initiation site for amplification, enhancing the overall efficiency of the LAMP reaction.

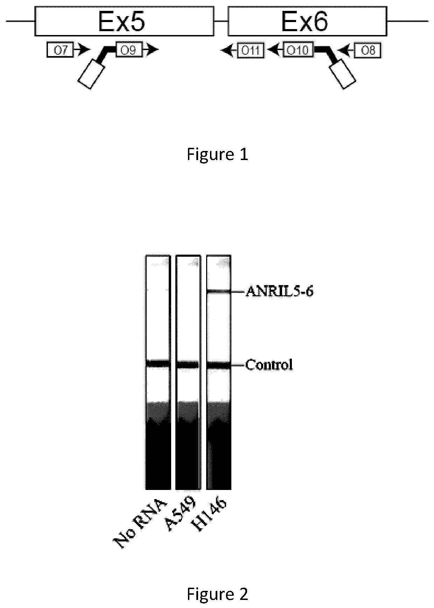

Their positions on exons 5 and 6 appears in Figure 1.

### 1.2. Guide RNA sequence for cleavage by Cas12a (IDT)

### CrRNA ex5-6 is as follows:

/AltR1/UAAUUUCUACUAAGUGUAGAUACAGUCUCUCCAAUGAA CGCC/AltR2/

### 1.3 Sequence Probe (IDT)

OProbe: 5'/6-FAM/TTATTATT/Bio/-3', HPLC purified

### 2. LAMP reaction

| **Component of the reaction mix** | **Detection of ANRIL Exons 5 and 6** | **No template control** |
|---|---|---|
| SuperScript IV RT-LAMP Master Mix (NEB Biolabs) | 12.5 µL | 12.5 µL |
| 40 µM primers O9 and O10 Mix | 1 µL | 1 µL |
| 10 µM primers O7 and O8 Mix | 0.5 µL | 0.5 µL |
| 10 µM primers O11 | 1 µL | 1 µL |
| Total RNA (10 ng/µL) | 1 µL | |
| Nuclease-free water | 9 µL | 10 µL |

| | | |
|---|---|---|
| Incubate for 15 min at 65°C Incubate for 2 min at 95°C | | |

### 3. Cas12a-mediated cleavage reaction

### Complex formation

| | Stock | Final | Dilution factor | 18 µL |
|---|---|---|---|---|
| Cas12a | 5 µM | 250 nM | 20 | 0.9 µL |
| O56 (guide RNA) | 6.25 µM | 62.5 nM | 100 | 0.18 µL |
| 10x NEB2.1 | 10x | 1x | 10 | 1.8 µL |
| H2O | | | | 15 µL |

### Incubate for 10 min at 37°C

### Add Probe

| | Stock | Final | Dilution factor | 18 µL |
|---|---|---|---|---|
| Reporter DNA OProbe | 50 µM | 500 nM | 100 | 0.18 µL |

### Prepare the following mixture

1 µL LAMP reaction (diluted 1/16)
18 µL Cas12a-RNA guide complex + Probe
81 µL 1X NEB2.1 buffer (Biolabs)

### Incubate for 20 min at 37°C

### 4. Detection (Milenia Hybridetect)

Mix 50 µL of cleavage reaction with 50 µL of Hybridetect (Milenia Hybridetect) Assay Buffer (1 :1) and incubate 5 min at room temperature (RT), which corresponds to about 20°C.

Place the HybriDetect Dipstick with the sample application into the solution mix and incubate 5 min at RT in an upright position.

Then remove dipsticks from the assay solution and interpret test result immediately.

### 5. Results obtained when applying the method of detection/quantification on A549 and H146 cell lines

As a proof of principle, we tested the method on 2 cell lines belonging to each subclass of lung cancer lines: A549 (NSCLC i.e. known to be of low aggressiveness) and H146 (SCLC i.e. known to be of high aggressiveness). We assumed that, since the two cell lines have drastic differences in aggressiveness, they must differentially express ANRIL-containing exons 5-6 isoforms to a significant extent. Therefore, in this scenario, a positive result is anticipated for the H146 and not for the A549 cell lines.

Interestingly, the results shown in Figure 2 are coherent with this assumption, as a band corresponding to the antibody line is observed on the strip in the context of the H146 cells, which is not the case for the negative control (without RNA) and the A549 cell line.

In conclusion, these results demonstrate that the detection of exons 5-6 of ANRIL allows the prediction of aggressiveness of cancer.

### Reference List

1. Cunningham et al: « Ensembl 2022 ». Nucleic Acids Research 50, no D1 (7 janvier 2022): D988 95.
2. Liu et al.: « High Expression of ANRIL Correlated with the Poor Prognosis in Patients with Cancer: A Meta-Analysis ». Medicine 101, no 36 (9 septembre 2022): e30531.
3. Vijay and Martin Filion. « New Developments in Quantitative Real-Time Polymerase Chain Reaction Technology ». Current Issues in Molecular Biology 16 (2014): 1-6.
4. Alfeghaly et al. « Implication of repeat insertion domains in the trans-activity of the long non-coding RNA ANRIL ». Nucleic Acids Research 49, n° 9 (21 mai 2021): 4954-70.
5. Soroka et al. « Loop-Mediated Isothermal Amplification (LAMP): The Better Sibling of PCR? » Cells 10, no 8 (29 juillet 2021): 1931.
6. Chen et al.: « CRISPR-Cas12a Target Binding Unleashes Indiscriminate Single-Stranded DNase Activity ». Science (New York, N.Y.) 360, no 6387 (27 avril 2018): 436 39.
7. Broughton et al. « CRISPR-Cas12-Based Detection of SARS-CoV-2 ». Nature Biotechnology 38, n° 7 (juillet 2020): 870-74.
8. Sun et al.: « One-Tube SARS-CoV-2 Detection Platform Based on RT-RPA and CRISPR/Cas12a ». Journal of Translational Medicine 19, n° 1 (16 février 2021): 74.
9. Paul and Montoya. « CRISPR-Cas12a: Functional Overview and Applications ». Biomedical Journal 43, n° 1 (février 2020): 8-17.
10. Sanchez et al. : « The Long Non-Coding RNA ANRIL in Cancers", Cancers 2023, 15, 4160. https://doi.org/10.3390/cancers15164160.

## Claims

1. Method of detecting and/or quantifying the presence of exons 5 and 6 of ANRIL (Antisense Non-coding RNA in the INK4 Locus) in a subject, comprising the following steps of:
1) Preparation of total RNA from a biological sample of the subject,
2) Conversion of RNA molecules into complementary DNA,
3) Loop-mediated isothermal amplification using trans-exonic oligonucleotides that specifically amplify exons 5 and 6,
4) Addition of Cas12a/RNA guide complexes, wherein RNA guide specifically targets exons 5 and 6 by base pairing,
5) Addition of a reporter DNA probe,
6) Detection of cleavage products, wherein detection of cleavage products reveals the presence of exons 5 and 6 of ANRIL, and
7) possibly quantifying expression of exons 5 and 6 of ANRIL.

2. Method according to claim 1, wherein said trans-exonic oligonucleotides that specifically amplify exons 5 and 6 are at least one chosen among:
- O7: TGTCCCTTTTGATGAGAAGA (SEQ ID NO: 1)
- O8: CCAAATAGATCTCCCCGG (SEQ ID NO: 2)
- O9: ACATATATCTGGTGGCCAGAAAACACCTCATTCTGATTCAACAGC (SEQ ID NO: 3)
- O10: AGGAGAATTTTCTTGGAAAGAGAGGAAAGCAGTACTGACTCGG (SEQ ID NO: 4)
- O11: CATCACTGTTAGGTGTGCTGGAAT (SEQ ID NO: 5).

3. Method according to claim 2, wherein said trans-exonic oligonucleotides are at least one chosen among:
- a mix of O9 (SEQ ID NO: 3) and O10 (SEQ ID NO: 4), and
- a mix of O7 (SEQ ID NO: 1) and O8 (SEQ ID NO: 2).

4. Method according to any one of the preceding claims, wherein said RNA guide has the sequence:
UAAUUUCUACUAAGUGUAGAUACAGUCUCUCCAAUGAACGCC (SEQ ID NO: 6).

5. Method according to any one of the preceding claims, wherein said reporter DNA probe is coupled 5' to 6-Carboxyfluorescein (F) and 3' to biotin (B).

6. Method according to claim 5, wherein said reporter DNA probe comprises at least one sequence chosen among the sequence TTATTATT, TTATT and TTATTATTATT (SEQ ID NO: 7).

7. Method according to any one of the preceding claims, wherein detection of cleavage products is carried out by anti-fluorescein antibodies.

8. Method for in vitro diagnosis of a cancer in a subject, comprising the step of:
- detecting the presence of exons 5 and 6 of ANRIL in a biological sample of a subject with a method as defined in any one of claims 1 to 7, Wherein:
- a detection of exons 5 and 6 of ANRIL in the sample indicates a presence of cancer, and,
- an absence of detection of exons 5 and 6 of ANRIL in the sample indicates an absence of cancer.

9. Method for in vitro prognosis a cancer in a subject, comprising the step of:
- quantifying expression of exons 5 and 6 of ANRIL in a biological sample of a subject with a method as defined in any one of claims 1 to 7, Wherein:
- A higher abundance level of exons 5 and 6 of ANRIL compared to a reference value is predictive to a more aggressive cancer, and
- An equivalent or lower level of exons 5 and 6 of ANRIL compared to a reference value is predictive of a less aggressive cancer.

10. Method according to claim 9, wherein the subject is suffering from a cancer chosen among lung, gastric, breast, ovarian, cervical, colorectal, bladder, thyroid, brain, osteosarcoma, myeloma, prostate, leukemia, melanoma, endometrial, renal, retinoblastoma, head/neck, intrahepatic cholangiocarcinoma and hepatocellular cancer.

11. Kit for implementing the method as defined in any one of claims 1 to 7, comprising the following elements:
- trans-exonic oligonucleotides for specific exons 5 and 6 amplification,
- RNA guides specifically targeting exons 5 and 6 by base pair pairing,
- a reporter DNA probe, and
- a mean for the detection of cleavage products.

12. Set of oligonucleotides having at least one sequence chosen among:
- O7: TGTCCCTTTTGATGAGAAGA (SEQ ID NO: 1)
- O8: CCAAATAGATCTCCCCGG (SEQ ID NO: 2)
- O9: ACATATATCTGGTGGCCAGAAAACACCTCATTCTGATTCAACAGC (SEQ ID NO: 3)
- O10: AGGAGAATTTTCTTGGAAAGAGAGGAAAGCAGTACTGACTCGG (SEQ ID NO: 4)
- O11: CATCACTGTTAGGTGTGCTGGAAT (SEQ ID NO: 5).

13. Set of oligonucleotides according to claim 12, chosen among:
- a mix of O9 (SEQ ID NO: 3) and O10 (SEQ ID NO: 4), and
- a mix of O7 (SEQ ID NO: 1) and O8 (SEQ ID NO: 2).

14. Use of a kit as defined in claim 11, or of a set of oligonucleotides as defined in claim 12 or 13, for detecting and/or quantifying exons 5 and 6 of ANRIL in a biological sample of a subject.
